# EUROPÄISCHE PATENTANMELDUNG

(11) **EP 1 947 461 A2**
(43) Veröffentlichungstag der Anmeldung: **23.07.2008**
(21) Anmeldenummer: 07121441.5
(22) Anmeldetag: 23.11.2007
(51) Int. Cl.: G01N 33/68, G01N 33/58, B01J 19/00, B01J 19/24, B01L 3/14

(54) **Verfahren zum quantitativen Vergleich von zwei oder mehr Proteinen**

(30) Priorität: 23.11.2006 DE 102006055358
(71) Anmelder: OMX GmbH, 82234 Wessling (DE)
(72) Erfinder: Eichacker, Lutz, 82234 Weßling (DE); Granvogl, Bernhard, 85716 Unterschleißheim (DE); Gruber, Patrick, 80336 München (DE)
(74) Vertreter: Fleuchaus, Michael A.

(57) **Zusammenfassung**

Die Erfindung betrifft ein Quantifizierungsverfahren, das auf der chemischen Umsetzung von Proteinen oder Peptiden mit Aceton und einem Hydrierungsreagenz (vorzugsweise Natriumcyanoborhydrid) basiert, wobei die Aminogruppen der Proteine bzw. Peptide reduktiv iso-propyliert werden. Durch Austausch von Aceton bzw. des Hydrierungsreagenzes mit entsprechenden stabilisotopenmarkierten Analoga ist es möglich, Proteine bzw. Peptide einer zweiten und dritten Probe mit Stabilisotopen in unterschiedlicher Zahl zu markieren. Nach Vereinigung der Proben lassen sich die ursprünglichen Zustände der Proteine bzw. Peptide massenspektrometrisch analysieren.

## Beschreibung

Die vorliegende Erfindung betrifft ein verbessertes Verfahren zur quantitativen Bestimmung von Peptiden und/oder Proteinen in zwei oder mehreren Proteingemischen.

Auf dem Gebiet der Proteomanalyse wird die Untersuchung, der Vergleich und die qualitative oder auch quantitative Analyse von komplexen Proteingemischen zunehmend wichtiger, denn es ist in den letzten Jahrzehnten durch die Entwicklung von neuen DNA Sequenzierungstechniken das Wissen über Genomstrukturen einschließlich der vollständigen Sequenzinformationen exponentiell angewachsen, und somit ist es von zunehmender Bedeutung Techniken zu entwickeln, die es erlauben die Korrelation zwischen der genetischen Information und der tatsächlichen biologischen Relevanz zu untersuchen. Derartige Fragen werden im Rahmen der Proteomanalyse gestellt, bei der häufig der Zustand bzw. der Unterschied zweier oder mehrerer biologischer Systeme verglichen und ausgewertet wird.

Ein Auswertungskriterium ist dabei der quantitative Vergleich der zu untersuchenden Proteingemische. Im Stand der Technik sind mehrere Verfahren zu quantitativen Bestimmung von Proteingemischen bekannt.

In WO 00/11208 und Gygi, S.P. et al. (Nature Biotechnology, 17, 1999, 994-999) wird ein Quantifizierungsverfahren beschrieben, bei welchem zwei getrennte Proteingemische I und II getrennt voneinander mit einem Reagenz versetzt werden, welches kovalent an bestimmte funktionelle Gruppen der Proteine bindet. Dieses Quantifizierungsverfahren bedient sich dabei der Reaktivität der Thiolgruppe von Cystein-Resten. Das Reagenz, welches dem Gemisch II zugesetzt wird, unterscheidet sich vom Reagenz, welches dem Gemisch I zugesetzt wird, nur durch eine Isotopenmarkierung, ist aber sonst chemisch identisch. Dabei kommen hauptsächlich Reagenzien, welche nichtradioaktive Isotope wie Deuterium oder Kohlenstoff-13 tragen, zum Einsatz. Die Proteingemische werden anschließend vereinigt und enzymatisch oder chemisch hydrolysiert. Das so entstandene Peptidgemisch wird massenspektrometrisch analysiert. Dabei werden bei Peptiden, welche mit dem Reagenz derivatisiert wurden, die Signalintensitäten der Isotopmere detektiert, da sich entsprechende Peptide aus den Gemischen I und II aufgrund der Isotopmarkierung in ihrer Molekülmasse unterscheiden. Das Signalverhältnis von nicht-markierten und markierten Peptidanaloga gibt das ursprüngliche Verhältnis der Proteingemische I und II wieder, so dass beide Proteingemische relativ zueinander quantifiziert werden können.

Ein Nachteil dieser Methode ist, dass die Aminosäure Cystein in Proteinen relativ selten auftritt (1 - 2 %) und so nur wenige Peptide erhalten werden, welche eine massenspektrometrische Quantifizierung erlauben.

Nach WO 00/11208 und Gygi, S.P. et al. (Nature Biotechnology, 17, 1999, 994-999) kann dieses Problem nicht umgangen werden, indem in das Derivatisierungsreagenz eine chemische Gruppe eingefügt wurde, welche sich durch eine besondere Affinität zu anderen Verbindungen auszeichnet. Dieses sog. Affinitätslabel erlaubt es lediglich, die derivatisierten Peptide aus dem komplexen Peptidgemisch selektiv zu isolieren. Beispielsweise kann das Derivatisierungsreagenz eine Biotin-Gruppe aufweisen. Die derivatvisierten Peptide können dann mittels reversibler Bindung an immobilisiertes Avidin oder Streptavidin isoliert werden.

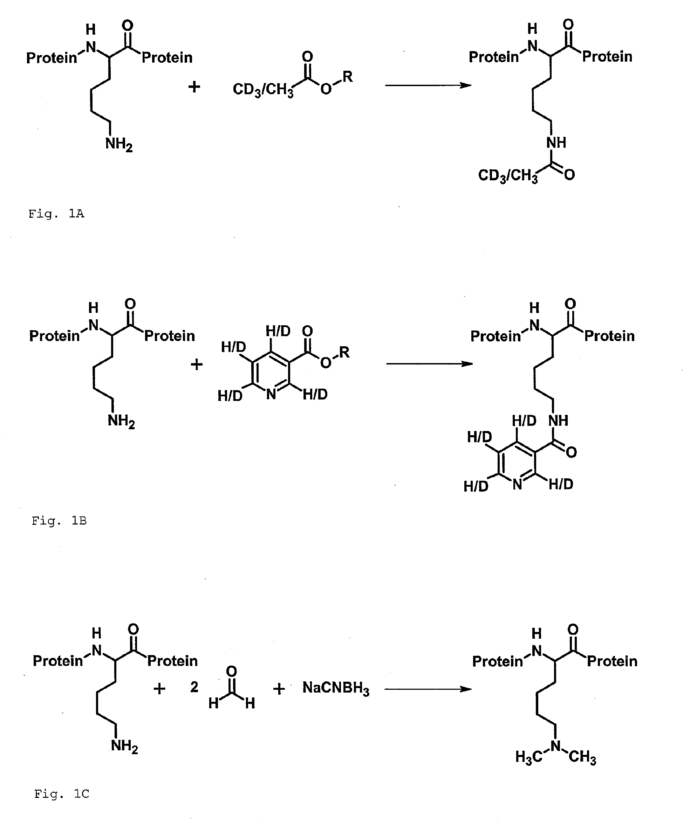

Andere Entwicklungen zur Einführung von Isotopenmarkierungen bedienen sich der Reaktivität von Aminogruppen, welche in Proteinen in den Lysinseitenketten und am N-Terminus vorkommen (Münchbach et al. (Anal. Chem., 2000, 72, 4047 - 4057)). Die Isotopenmarkierung kann wahlweise vor oder nach Hydrolyse der Proteingemische durchgeführt werden. Als Reagenzien haben vor allem aktivierte Carbonsäuren Verbreitung gefunden, welche die Aminogruppen in Acylamide überführen. Als aktivierte Carbonsäuren kommen hauptsächlich Carbonsäureanhydride, - halogenide und -aktivester zum Einsatz. Weite Verbreitung hat dabei die Essigsäure in Form ihres Anhydrids bzw. NHS-Esters gefunden (Fig. 1A), da die entsprechenden stabilisotopenmarkierten Analoga kommerziell erhältlich und relativ preisgünstig sind (Ji et al., J. Chromatogr. B, 745, 2000, 197-210). Andere stabilisotopenmarkierte Carbonsäuren bzw. ihre aktivierten Derivate sind meist teuer und/oder nicht kommerziell verfügbar.

Ein Nachteil dieser Methode ist, dass die Proteine bzw. Peptide durch Acylierungsreaktionen einen Teil ihrer Ladungsträger verlieren, da aus einer protonierbaren Aminogruppe eine nichtprotonierbare Säureamidbindung entsteht. Die Ionisierbarkeit eines Peptides ist jedoch eine Grundvoraussetzung zur massenspektrometrischen Analyse. Die wichtigsten Ionisierungsmethoden für Peptide basieren auf der Bildung von positiv geladenen Pseudomolekülionen durch Adduktbildung mit positiven Ladungsträgern, vornehmlich Protonen (H⁺). Da N-acylierte Peptide einen Teil ihrer protonierbaren Gruppen verlieren, sinkt die Ionisierungsrate der Peptide, was zu einer verminderten Empfindlichkeit in der massenspektrometrischen Analyse führt. Darüber hinaus weisen N-Acylpeptide eine hohe Affinität zu Alkalimetallionen auf, so dass sich im Massenspektrum neben protonierten Peptiden auch die Signale von Pseudomolekülionen mit Alkalimetallen, wie Natrium, finden. Dies führt zu sehr komplexen Massenspektren, deren Interpretation deutlich erschwert ist (Ji et al., J. Chromatogr. B, 745, 2000, 197-210).

Münchbach et al. (Anal. Chem., 2000, 72, 4047 - 4057) und DE 101 53 631 (Lottspeich et al.) beschreiben Verfahren, bei denen die zu quantifizierenden Proteingemische jeweils mit ionisierbaren Labeln versehen werden und massenspektroskopisch analysiert werden. Die oben erwähnten Probleme der Ionisierbarkeit werden in Münchbach et al. (Anal. Chem., 2000, 72, 4047 - 4057) und DE 101 53 631 umgangen, indem Derivatisierungsreagenzien der Struktur R-PRG verwandt werden, wobei die proteinreaktive Gruppe (PRG) einen Rest (R) trägt, welcher mindestens eine positive oder negative Elementarladung enthält (Fig. 1B). Ein Beispiel für eine derartige Verbindung ist eine aktivierte Nicotinsäure (Münchbach et al. (Anal. Chem., 2000, 72, 4047 - 4057). Bei Umsetzung mit Aminogruppen wird zwar die Protonierbarkeit der Aminogruppe durch Bildung einer Aminbindung aufgehoben, gleichzeitig aber ein protonierbarer Nikotinsäurerest eingefügt, so dass der Ladungszustand im derivatisierten Peptid nicht verändert wird. Nachteil dieser Methode ist der hohe Preis von stabilisotopenmarkierten Analoga derartiger Reagenzien.

Hsu et al (Anal. Chem., 2003, 75,6843-6852) beschreiben eine weitere Möglichkeit zum Erhalt der Protonierbarkeit beruhend auf der Reaktion von Aminogruppen mit Formaldehyd in Gegenwart von Natriumcyanoborhydrid (Fig. 1C). Dabei werden peptidische Aminogruppen durch reduktive Alkylierung in Dimethylaminogruppen überführt. Aus dem primären Aminogruppen der N-Termini bzw. den Lysinseitenketten von Proteinen und Peptiden entstehen so tertiäre Amine, welche protonierbar bleiben.

Ein Nachteil dieser Methode ist, dass Formaldehyd äußerst reaktiv ist und neben primären Aminogruppen auch andere funktionelle Gruppen von Proteinen modifizieren kann. So kann es beispielsweise zu Modifizierungen von Tryptophanresten oder zu Crosslinks kommen (Metz et al., J. Biol. Chem., 279, 8, 2004, 6235-6243; Fu & Li, J. Am. Soc. Mass Spec., 17, 2006, 859-866).

Ein weiterer Nachteil ist die Giftigkeit von Formaldehyd, welches als potentiell krebserregend eingestuft wird. Darüber hinaus ist Formaldehyd eine instabile Verbindung, die bei Lagerung Zersetzungs-, Oxidations- und Polymerisierungsreaktionen unterliegt.

Die beim Formaldehyd beschriebe Unspezifität der Reaktion beruht auf der großen Reaktivität des Formaldehyds, da diese Verbindung das einfachste und daher reaktivste Aldehyd darstellt. Hierdurch erklären sich die Nebenreaktionen mit anderen funktionellen Peptidgruppen wie die Hydroxymethylierung von Tryptophanresten oder die Ausbildung von Quervernetzungen von Peptiden bzw. Proteinen.

Es war daher Aufgabe der vorliegenden Erfindung ein alternatives, verbessertes und/oder günstigeres Quantifizierungsverfahren bereit zustellen, bei dem keine teuren Reagenzien, keine instabilen oder krebsverdächtigen Chemikalien und auch keine unspezifisch reagierenden Agenzien eingesetzt werden.

Um die Spezifität eines Peptidlabeling auf Basis einer reduktiven Alkylierung von peptidischen Aminogruppen zu erhöhen, war es Gegenstand der vorliegenden Erfindung, das hochreaktive Aldehyd Formaldehyd gegen eine reaktionsträgere Oxo-Verbindung auszutauschen.

Die Aufgabe wird durch das Verfahren gemäß Anspruch 1 gelöst, wobei weitere bevorzugte Ausführungsformen in den Unteransprüchen beschrieben werden.

In ihrer einfachsten Form stellt die vorliegende Erfindung ein Quantifizierungsverfahren für zwei Protein- oder Peptidproben dar, wobei in einer Derivatisierungsreaktion die eine Protein- oder Peptidprobe durch reduktive Alkylierung mit einem Keton, vorzugsweise Aceton, und einem Hydrierungsreagenz z. B. am N-terminalen Ende und/oder an Aminogruppen von Seitenketten von Aminosäuren (z. B. Lysin, Ornithin) derivatisiert werden. Die andere Probe wird mit isotopenmarkierten Varianten des Ketons und/oder mit der isotopenmarkierten Variante des Hydrierungsreagenzes umgesetzt.

Ketone gelten im Allgemeinen als reaktionsträge verglichen mit Aldehyden und sind daher besonders geeignet die Probleme des Standes der Technik zu lösen. Diese Reaktionsträgheit wird durch die Anwesenheit von zwei Alkylgruppen in Nachbarschaft zur Oxo-Funktion erklärt, da durch deren (+)I-Effekt die Polarität der C=O-Bindung geschwächt wird.

Zunächst bildet das eingesetzte Keton mit der proteinischen oder peptidischen Aminogruppe unter Wasseraustritt ein Imin, welches dann unter Einfluss des Hydrierungsreagenzes zu einem Amin reduziert wird.

Es wurde nun überraschenderweise gefunden, dass die reduktive Alkylierung von Peptiden z. B. mit Aceton und Natriumcyanoborhydrid hochselektiv für Aminogruppen ist. Nebenreaktionen wie Modifizierungen von Tryptophanresten oder Quervernetzungen wurden nicht beobachtet. Da Aceton reaktionsträger ist als Formaldehyd, benötigt die vollständige Reaktion von Peptiden mit Aceton/Natriumcyanoborhydrid mehr Zeit als die Reaktion mit Formaldehyd/Natriumcyanoborhydrid. Während sich letztgenannte Reaktion binnen Minuten vollzieht, benötig die Umsetzung mit Aceton mehrere Stunden.

Es zeigte sich, dass bei der erfindungsgemäßen Alkylierung mit Aceton nur ein iso-Propylrest an einer Aminogruppe eingeführt wird und dadurch nur ein sekundäres Amin entsteht. Eine Weiterreaktion zu einem tertiären Amin und wird dabei durch die sterische Änderung des iso-Propylrests verhindert. Durch den Einbau einer iso-Propylgruppe verändert sich die Masse des derivatisierten Proteins oder Peptids um 42 Da.

Zum quantitativen Vergleich zweier Proteinproben können unterschiedliche Isotopomere des Ketons, insbesondere des Acetons, und/oder stabilisotopenmarkierte Varianten des Hydridüberträgers eingesetzt werden.

Gemäß einer Ausführungsform ist der vorzugsweise im erfinderischen Verfahren eingesetzte Hydridüberträger das Hydrierungsreagenz Natriumcyanoborhydrid bzw. Natriumcyanobordeuterid. Unter Verwendung von z. B. Natriumcyanoborhydrid bzw. Natriumcyanobordeuterid werden in der Reduktion selektiv nur Imine und keine anderen funktionellen Gruppen im Protein (vor allem Amide) reduziert.

Besonders hervorzuheben sind die vorteilhaften Eigenschaften des zur Derivatisierung eingesetzten Ketons, vorzugsweise des Acetons, welches insbesondere im Vergleich zu dem üblicherweise verwendeten Formaldehyd folgende Vorteile zeigt:
- Aceton ist im Gegensatz zu Formaldehyd stabil und deutlich weniger gesundheitsschädlich.
- Die Derivatisierungsreaktion mit Aceton ist für Aminogruppen spezifisch und gezielt steuerbar, während Formaldehyd unspezifisch alkyliert und z. B. auch Aminosäuren wie Tryptophan modifiziert.
- Aufgrund seiner sterischen Eigenschaften führt Aceton lediglich eine iso-Propylgruppe bei der Alkylierung ein, während es mit Formaldehyd zu einer Doppelalkylierung kommt.
- Stabilisotopenmarkiertes D6-Aceton ist äußerst kostengünstig und durchschnittlich um einen Faktor 10 günstiger als das Mol stabilisotopenmarkiertes Formaldehyd.
- Stabilisotopenmarkiertes D6-Aceton führt pro Derivatisierungsstelle 6 Deuteriumatome ein, was zu einem Massenshift von 6 Da im Vergleich zur mit H6-Aceton behandelten Probe führt. Dadurch sind die Signale der Isotopomere auch bei großer Molekülmasse gut abgetrennt. Bei den Verfahren mit Formaldehyd bzw. aktivierter Essigsäure werden nur 4 bzw. 3 Deuteriumatome eingeführt.

Eine Derivatisierung mit einem Keton, vorzugsweise Aceton, zeichnet sich weiterhin dadurch aus, dass die Reaktion im Gegensatz zum sehr reaktiven Formaldehyd langsamer voranschreitet und sich dadurch chemoselektiv steuern lässt. So kann z. B. über die Wahl eines geeigneten pH-Wertes die N-terminale Aminogruppe eines Peptides selektiv alkyliert werden, während die Aminogruppen in den Seitenketten von Aminosäuren nicht derivatisiert werden.

Ferner ermöglicht es dieses Verfahren, den ursprünglichen Ladungszustand der Aminogruppen beizubehalten, so dass die derivatisierten Produkte massenspektroskopisch ohne Adduktbildung detektierbar sind.

Bei Verwendung von D6-Aceton als stabilisotopenmarkiertes Reagenz werden bei jeder iso-Propylierung 6 Deuteriumatome in die Probe eingebaut. Die chemisch gleichen Peptide der zu vergleichenden Proben unterscheiden sich folglich nur durch die Anzahl der eingebauten Deuteriumatome. Jede iso-Propylierung, die 6 Deuteriumatome einbaut, erhöht die Masse des Peptides um weitere 6 Da. Eine iso-Propylierung einer Aminogruppe mit Aceton führt folglich zu einer Massenerhöhung von 42 Da und eine iso-Propylierung einer Aminogruppe mit stabilisotopenmarkierten D6-Aceton führt zu einer Massenerhöhung von insgesamt 48 Da (vgl. Figur 5 und Figur 6).

Wie oben beschrieben führt eine iso-Propylierung zu einer Massenzunahme von 42 Da. Den gleichen Massenshift beobachtet man auch bei Acetylierungen mit aktivierter Essigsäure. Iso-Propylierungen und Acetylierungen führen also zu isobarischen Peptiden. Allerdings führt man bei Verwendung von D6-Aceton sechs Deuteriumatome pro Modifizierung ein, während bei Verwendung von aktivierter D3-Essigsäure nur 3 Deuteriumatome eingeführt werden. Dadurch unterscheiden sich - bei einer Modifizierungsstelle - nicht-markiertes und markiertes Peptid bei der iso-Propylierung um 6 Da, bei der Acetylierung um 3 Da. Dieser Effekt wirkt sich positiv bei der Quantifizierung von großen Peptiden oder Proteinen aus.

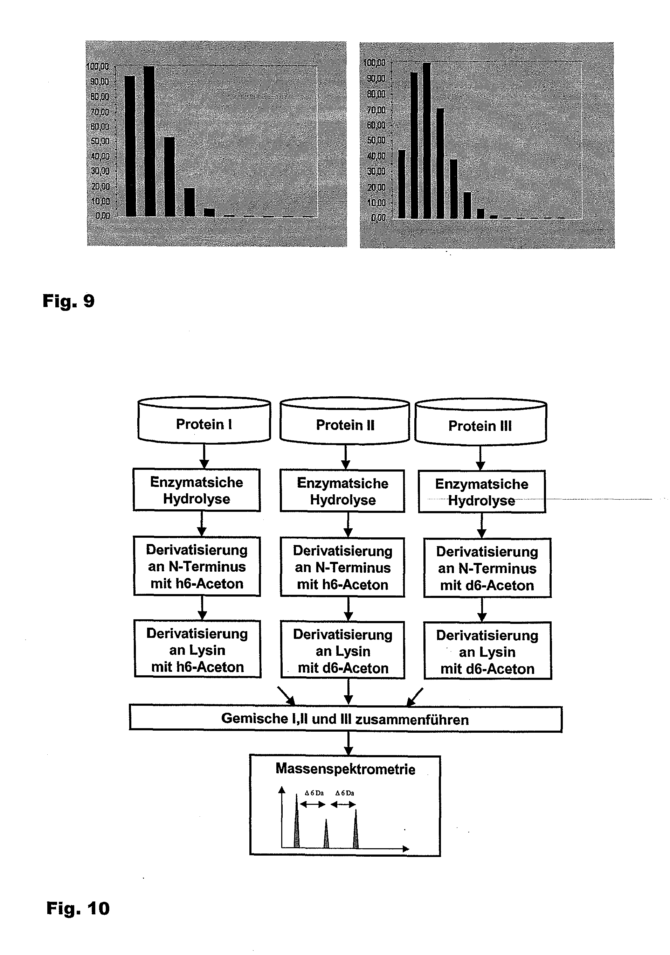

Aufgrund der natürlichen Häufigkeit von Deuterium und auch Kohlenstoff-13 (13C) zeigen Peptide ab einer Masse von ca. 1500 Da ein Isotopomerenmuster, bei dem das monoisotopische Signal vom ersten Isotopomerenpeak (1 Deuterium bzw. 13C) überragt wird. Ab ca. 3000 Da wird der zweite Isotopomerenpeak zum höchsten Signal (Fig. 9). Die Intensitäten höherer Isotopomere steigen dabei sukzessive an.

Bei einem Labeling mit nur 3 Deuteriumatomen durch aktivierte D3-Essigsäure bzw. 4 Deuteriumatomen durch Umsetzung mit deuteriertem Formaldehyd würden sich die Signale von nicht-markiertem und markiertem Peptid überlagern, so dass eine Quantifizierung ohne mathematische Behandlung der Messsignale unmöglich wird. Werden jedoch 6 Deuteriumatome eingebaut, so rücken die Signale von nicht-markiertem und markiertem Peptid so weit auseinander, dass keine Überlagerung auftritt.

Da sich diese Masseverschiebung im Massenspektrogramm gut darstellen, ist das vorliegend beschriebene Verfahren ideal geeignet, auch drei oder mehr Protein- oder Peptidproben quantitativ zu analysieren und zueinander in Verhältnis zu setzen.

Das erfindungsgemäße Verfahren erlaubt daher insbesondere auch die quantitative Analyse von drei Peptid- oder Proteinproben. Hierzu werden die drei Proben zunächst einer ersten Derivatisierungsreaktion unterzogen, bei der zwei der Proben mit dem Keton, vorzugsweise Aceton, und eine der drei Proben mit einem Keton, vorzugsweise stabilisotopenmarkierten Aceton, an der N-terminalen Aminogruppe iso-propyliert werden.

In der zweiten Derivatisierungsreaktion wird dann die erste Probe erneut mit dem Keton, vorzugsweise Aceton, und die zweite und dritte Probe mit dem stabilisotopenmarkierten Keton, vorzugsweise D6-Aceton, umgesetzt. Bei diesem zweiten Schritt wird die Reaktion so gesteuert, dass nun die Aminogruppen der Seitenketten, vorzugsweise an den Lysinresten, iso-propyliert werden.

Gemäß der vorliegenden Erfindung umfasst das Quantifizierungsverfahren auch den Schritt der enzymatischen Hydrolysierung der zu quantifizierenden Proteinproben. Dieser Hydrolysierungsschritt kann vor, nach oder zwischen den Derivatisierungsreaktionen durchgeführt werden. Geeignete Enzyme zur Hydrolysierung sind z. B. Trypsin, Lys-C und Arg-C.

Die vorliegende Erfindung umfasst auch die quantitative Analyse von vier und/oder mehr Protein-und Peptidproben. Hierzu werden die einzelnen Proben, jede für sich, in einer individuellen Reihenfolge bzw. mit unterschiedlichen Reagenzien, die ebenfalls in individueller Reihenfolge eingesetzt werden, behandelt.

Neben dem erfindungsgemäßen Keton, vorzugsweise Aceton, und stabilisotopenmarkiertem Aceton (D6-Aceton) ist auch die Verwendung von Essigsäureanhydrid und stabilisotopenmarkierte Essigsäureanhydrid (D3-AcOR) geeignet.

Um nun vier oder mehr Protein- oder Peptidproben zu quantifizieren, wir hierin eine sequenzielle Markierung bzw. ein sogenanntes sequenzielles Labelling vorgeschlagen. Bei einem sequenziellen Markieren werden dabei z. B. in einem ersten Schritt die Lysinseitenketten des intakten Proteins mit einer beliebigen oder vorgeschlagenen Derivatisierungsreagenzie behandelt, wobei sowohl markierte wie nicht-markierte Verbindungen eingesetzt werden. Hieran schließt sich dann z. B. eine enzymatische Hydrolyse, so dass in einer zweiten Derivatisierungsreaktion die peptidisch entstandenen N-Termini mit einer weiteren der vorgeschlagenen Derivatisierungsreagenzien behandelt werden.

Um in einem Quantifizierungsverfahren vier Proben zu analysieren, können beispielsweise in einem ersten Derivatisierungsschritt die Probe I und III mit aktivierten H3-Essigsäure und die Probe II und IV mit aktivierten D3-Essigsäure umgesetzt werden. Nach einer enzymatischen Hydrolysierung wird dann die Probe I und II mit H6-Aceton und die Probe III und IV mit D6-Aceton behandelt. Es entstehen dabei chemisch gleiche Peptide, die sich nur in der Anzahl der Deuteriumatome unterscheiden. Bei der eben beschriebenen sequenziellen Markierung enthält das Peptid der Probe I kein Deuterium, das Peptid der Probe II enthält 3 Deuteriumatome, das Peptid der Probe II enthält 6 Deuteriumatome und das Peptid der Probe IV enthält 9 Deuteriumatome. Entsprechend kann mittels Massenspektroskopie das ursprünglich quantitative Verhältnis der Proteine der vier Proben durch Vergleich der Signalintensitäten ermittelt werden.

Die Reihenfolge der Derivatisierungen mit Aceton und/oder Essigsäure sind dabei variierbar, um zu gewährleisten, dass die entstehenden Peptide verschieden viele Deuteriumatome enthalten.

Bei Quantifizierungsverfahren von 4 und mehr Proben ist darauf zu achten, dass die Proben zwischen den einzelnen Derivatisierungsschritten gut gewaschen werden, da ansonsten eventuell das falsche Reagenz eine durch Verdau neu entstandene Aminogruppe derivatisiert.

In einer weiteren Ausführungsform umfasst das hierin beschriebene Verfahren auch einen Verfahrensschritt zur Trennung von komplexen Proteinproben. Geeignete Abtrennungsverfahren sind z. B. die ein- oder zweidimensionalen Gelelektrophorese oder verschiedene chromatographische Trenntechniken.

Der Trennungsschnitt kann vor, nach oder zwischen den Derivatisierungsreaktionen oder alternativ auch vor oder nach der enzymatischen Hydrolyse durchgeführt werden. Er ist ferner nicht zwingend sondern optional.

Weiterhin umfasst das hierin beschriebene Verfahren den Schritt der Probenzusammenfassung, um dann die verschiedenen vorbehandelten, d. h. mit verschiedenen Reagenzien oder in verschiedener Reihenfolge derivatisierten oder hydrolysierten Proben in einer massenspektroskopischen Messung zu untersuchen. Auch dieser Schritt ist nicht zwingend, sondern optional, da die einzelnen Proben auch getrennt im Massenspektrometer analysiert werden könnten.

Da das hierin beschriebene Verfahren darauf aufbaut, dass die Aminogruppen von z. B. Lysinresten bzw. Ornithinresten derivatisiert werden, ist darauf zu achten, dass Lysin-freie Proteine oder Peptide bzw. Proteine oder Peptide mit mehrfachen Lysinresten abweichende Isotopomermuster zeigen.

Die vorliegende Erfindung stellt ferner in einer weiteren Ausführungsform ein Verfahren bereit, das durch die Wahl der Reaktionsbedingungen in der Derivatisierungsreaktion die Erhöhung der Spezifität der reduktiven Alkylierung ermöglicht. So ist gemäß einer Ausführungsform die vorrangige bis ausschließliche Derivatisierung der N-terminalen Aminogruppen gewährleistet, sofern die Reaktion bei einem pH-Wert von ≤ 5 vorzugsweise einen pH-Wert von 1,0 - 2,0, 1,0 - 3,0, 2,0 - 3,0, 2,0 - 4,0, 3,0 - 4,0, 3,0 - 4,5 oder 3,5 - 5,0 durchgeführt wird.

Gemäß einer weiteren Ausführungsform wird die vorrangige bis ausschließliche Derivatisierung der anderen nicht-N-terminalen Aminogruppen an den Aminosäureseitenketten erreicht, indem die Reaktion bei einem pH-Wert von ≥ 5, vorzugsweise 5,0 - 6,0, 6,0 - 7,0, 6,0 - 8,0, 7,0 - 8,0 oder 8,0 - 9,0 durchgeführt wird.

Gemäß einer weiteren Ausführungsform kann das Verfahren auch gekoppelt an eine Festphase oder Matrix durchgeführt werden, wobei die erste und/oder zweite Derivatisierungsreaktion und/oder Hydrierungsreaktion der Peptid- oder Proteinprobe, an oder in eine Festphase oder Matrix gebunden oder zurückgehalten (retardiert oder temporär retardiert), stattfinden.

Im Rahmen dieser Anmeldung ist unter Festphase oder Matrix ein chemisch vernetztes oder quervernetztes Molekül oder Polymer zu verstehen, das aufgrund seiner Struktur in der Lage ist ein wässriges und/oder organisches Lösungsmittel aufzunehmen. Eine derartige Aufnahme erfolgt entweder durch Quellung oder nicht quellend, wobei die Martix dann benetzbar ist oder mit dem Lösungsmittel in Verbindung treten kann, wobei die Wechselwirkungen zwischen Matrix und Lösungsmittel polarer oder unpolarer Natur sein können. Eine für das vorliegende Verfahren geeignete Festphase oder Matrix ist z. B. ein Sorbens, vorzugsweise ein Ab-, Ad- oder Chemosorbens, weiter vorzugsweise Sepharose, Sephadex, Agarose, Acrylamid, Cellulose, Stärke, Kieselgel, modifiziertes Kieselgel oder ein Ionenaustauscher.

Sofern im Rahmen des vorliegenden Verfahrens die chemischen Umsetzungsreaktionen, nämlich die Derivatisierungs- und Hydrierungsreaktionen, an den Protein- oder Peptidproben, die an oder in einer Festphase oder Matrix gebunden oder zurückgehalten sind, durchgeführt werden, gelangen die Derivatisierungs- und Hydrierungsreagenzien durch einen Druckunterschied, der vor und nach (über und unter) der Festphase herrscht in die Festphase bzw. Matrix. Darüber hinaus können Kräfte, z.B. Kapilliarkräfte oder Zentrifugalkräfte eingesetzt werden, um das Eindringen der Derivatisierungs- und Hydrierungsreagenzien in die Festphase oder Matrix zu beschleunigen.

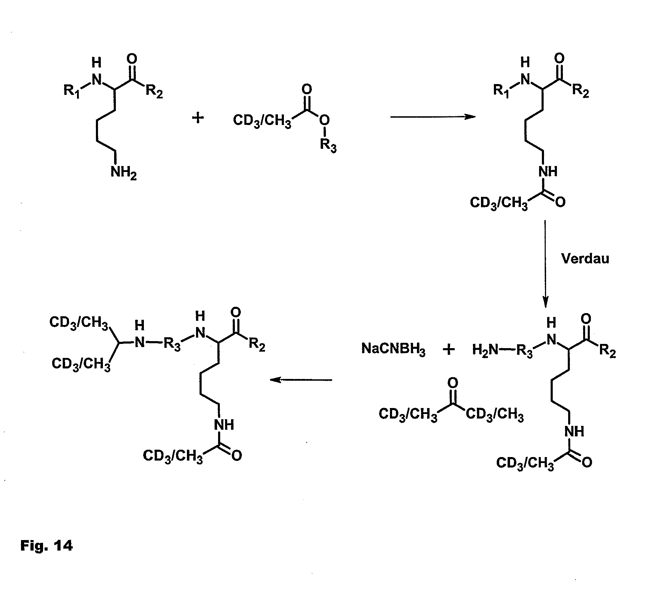

Gemäß einer weiteren Ausführungsform (Fig. 14) umfasst die vorliegende Anmeldung eine Reaktionseinheit bzw. ein Reaktionsgefäß zur Durchführung des Verfahrens zum quantitativen Vergleich von zwei oder mehr Protein- oder Peptidproben, wobei das Reaktionsgefäß einen ersten Reaktionsraum 1 aufweist, welcher der Aufnahme der Protein- oder Peptidprobe, z.B. in Lösung 2 oder in einer Matrix-Struktur eines Trägermaterials enthalten, dient und einen zweiten Reaktionsraum 3 aufweist, welcher der Aufnahme der Protein- oder Peptidprobe zur Durchführung wenigstens der ersten und/oder der zweiten Derivatisierungsreaktion und/oder Hydrolysierungsreaktion dient und einen dritten Reaktionsraum 4 aufweist, welcher der Aufnahme der chemisch umgesetzten Protein-oder Peptidprobe dient. Hierbei ist der erste Reaktionsraum mit dem zweiten Reaktionsraum durch einen Übergangsbereich 5 verbunden, der einen sich verengenden Querschnitt aufweist, welcher durch wenigstens ein bezüglich einer Längsachse der Reaktionseinrichtung zumindest teilweise geneigt verlaufendes Begrenzungselement bestimmt wird, und der zweite Reaktionsraum mit dem dritten Reaktionsraum durch einen Übergangsbereich verbunden ist, der derart ausgeführt ist, dass eine Festphase oder Matrix 6, in oder an welcher wenigstens die erste und/oder die zweite Derivatisierungsreaktion und/oder die Hydrolysierungsreaktion durchgeführt wird, in dem Übergangsbereich angeordnet ist.

Die Reaktionseinrichtung (Fig 14) der vorliegenden Erfindung zeichnet sich weiterhin dadurch aus, dass die Elemente, welche die einzelnen Reaktionsräume darstellen, voneinander abtrennbar, wieder zusammensetzbar, austauschbar und mit einem Deckel 8 verschließbar konstruiert sind.

Gemäß einer Ausführungsform ist die Reaktionseinrichtung eine Weiterentwicklung der bekannten OMX-S (erhältlich bei OMX GmbH, Deutschland) Reaktionsgefäße.

Die hiermit bereitgestellte Reaktionseinheit besteht aus einer Aufnahmevorrichtung 7, einer ersten Reaktionskammer 1, einem zweiten Reaktionsraum 3, in welchem eine Festphase bzw. Matrix 6 enthalten ist z. B. aus Säulenmaterial und einer dritten Reaktionskammer 4, in der die chemisch umgesetzten Endprodukte aufgefangen werden.

Gemäß dieser Ausführungsform ist die Aufnahmevorrichtung geeignet, um ein Stück, enthaltend die Proteinprobe aus einem Elektrophoresegel, aufzunehmen, welches dann mittels Zentrifugation in die erste Reaktionskammer gelangt.

Weiterhin ist die Aufnahmevorrichtung aber auch dazu geeignet, um eine flüssige Protein- oder Peptidprobe aufzunehmen, die dann mittels Zentrifugation oder aufgrund von Gravitation in den ersten Reaktionsraum läuft.

Weiterhin können auch Reaktionslösungen, zum Beispiel die Hydrolysierungsreagenzien oder die Derivatisierungsreagenzien in die Aufnahmevorrichtung pipettiert werden, um dann in dem ersten Reaktionsraum z. B. durch Zentrifugation überführt zu werden.

Je nach Verfahrensablauf findet im ersten Reaktionsraum entweder eine Derivatisierung oder Hydrolysierung statt.

Die chemisch umgesetzte Protein- oder Peptidprobe kann dann durch Umdrehen der Reaktionseinheit und z. B. Zentrifugation in den zweiten oder auch dritten Reaktionsraum überführt werden.

Gemäß einer Ausführungsform kann die Protein- oder Peptidprobe im zweiten Reaktionsraum z. B. an eine Festphase, z. B. eine Säule, gebunden, derivatisiert (acetyliert, acyliert, alkyliert, deacetyliert und/oder reduziert) werden.

Gebunden an diese Festphase, Matrix oder Säule kann die Probe auch gewaschen werden, wobei die Waschlösungen in dem dritten Reaktionsraum gesammelt werden. Anschließend wird der dritte Reaktionsraum gereinigt oder durch einen neuen (sterilen) ersetzt und die fertig umgesetzte Probe in diesem dritten Reaktionsraum aufgefangen.

### Figurenbeschreibung

Figur 1A zeigt die Umsetzung einer Lysinseitenkette mit aktivierter H3- bzw. D3-Essigsäure.
Figur 1B zeigt die Umsetzung einer Lysinseitenkette mit aktivierter H4- bzw. D4-Nicotinsäure.
Figur 1C zeigt die Umsetzung einer Lysinseitenkette mit Formaldehyd und Natriumcyanoborhydrid zu N^{ε}, N^{ε}-Dimethyllysin.

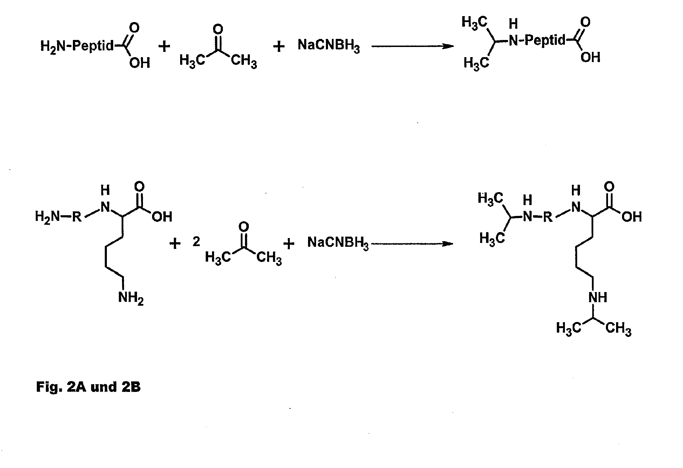

Figuren 2A und 2B zeigen die Umsetzung von Peptiden mit Aceton und Natriumcyanoborhydrid zu N^{α}-iso-Propylpeptiden (A) und N^{α}-iso-Propyl-(N^{ε}-iso-propyllysinyl)-peptiden (B).
Figur 3 zeigt die Umsetzung von Peptiden mit D6-Aceton und Natriumcyanoborhydrid zu N^{ε}-D6-iso-propyllysinyl)-peptiden).

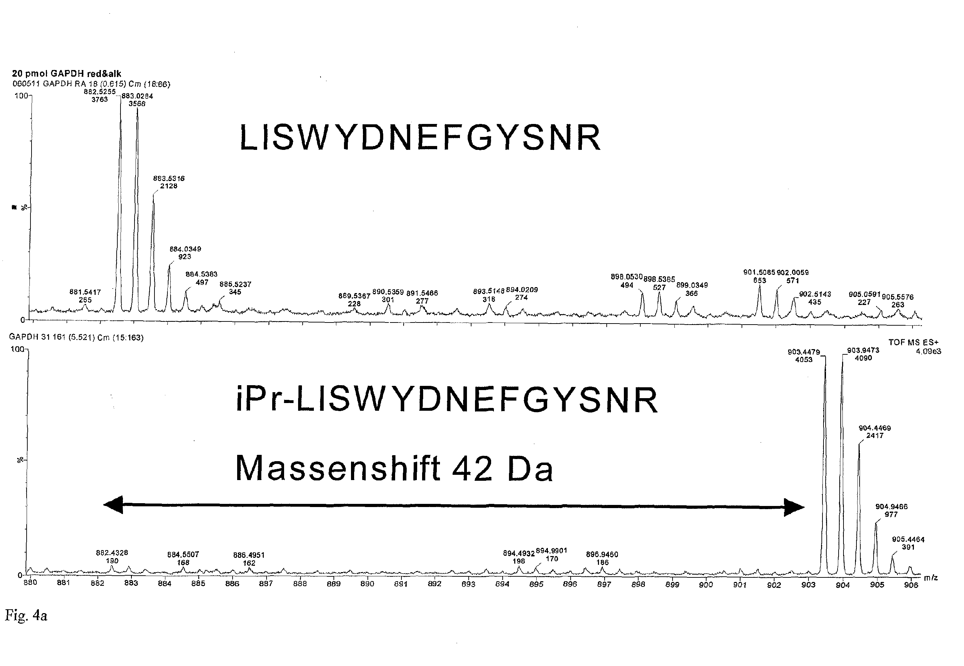

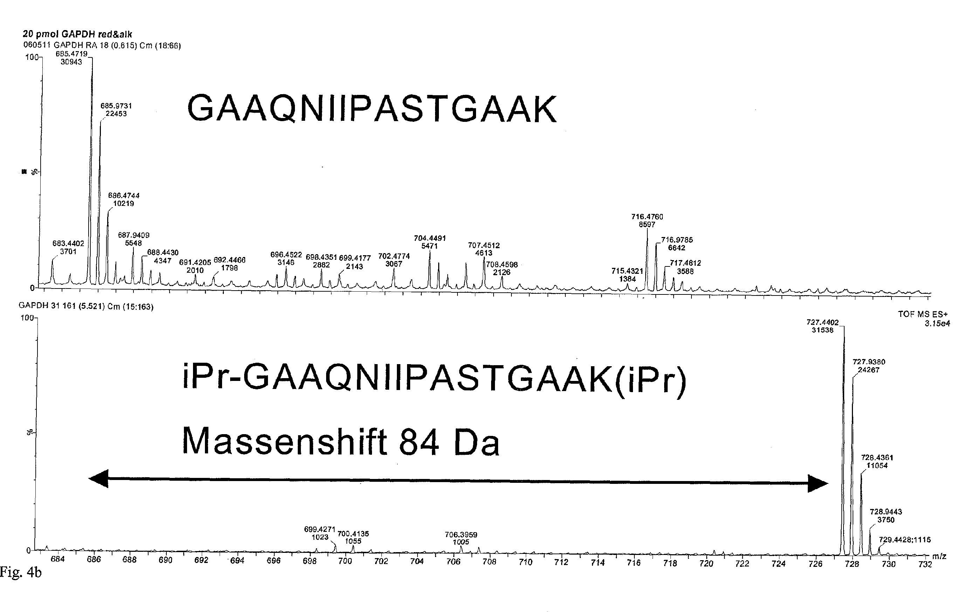

Figur 4 zeigt ESI-Massenspektren von Peptiden vor und nach Umsetzung mit Aceton und Natriumcyanoborhydrid.
Figur 5 zeigt ESI-Massenspektren von Peptiden vor und nach Umsetzung mit H6-Aceton bzw. D6-Aceton und Natriumcyanoborhydrid.
Figur 6 zeigt die graphische Darstellung der Intensität der Messsignale aus Beispiel 3.
Figur 7 zeigt ESI-Massenspektren von Mischungen (1:3 bis 3:1) eines nicht-markierten und markierten Peptides aus einem tryptischen Verdau.
Figur 8 zeigt den Vergleich von ESI-Massenspektren eines tryptischen Verdaus von GAPDH unbehandelt (oben), nach Acetylierung (Mitte), und nach iso-Propylierung (unten, 1:1 Mischung aus einer H6- und D6-iso-Propylierung). Nach Acetylierung verschwindet das Signal des Peptides. Acm = Amidocarboxymethyl (Schutzgruppe für Cysteinreste).
Figur 9 zeigt die Simulation der MS-Signale eines hypothetischen Peptides der Masse 1500 Da bzw. 3000 Da.
Figur 10 zeigt eine schematische Darstellung eines quantitativen Vergleichs von 3 Proteinzuständen mit H6/D6-iso-Propylierung an unterschiedlichen Aminogruppen von Peptiden.
Figur 11 zeigt ESI-Massenspektren von verschiedenen Peptiden eines tryptischen Verdaus nach H6/D6-iso-Propylierung analog. Es entstehen Isotopomere mit 6 bzw. 12 Da Massendifferenz.
Figur 12 zeigt eine schematische Darstellung einer Variante eines quantitativen Vergleichs von 3 Proteinzuständen mit H6/D6-iso-Propylierung an unterschiedlichen Aminogruppen von Peptiden.
Figur 13 zeigt eine schematische Darstellung eines quantitativen Vergleichs von 4 Proteinzuständen mit sequenzieller Markierung.
Figur 14 zeigt die Acetylierung von Proteinen, gefolgt von enzymatischer Hydrolyse und iso-Propylierung der entstehenden Peptid-N-Termini.

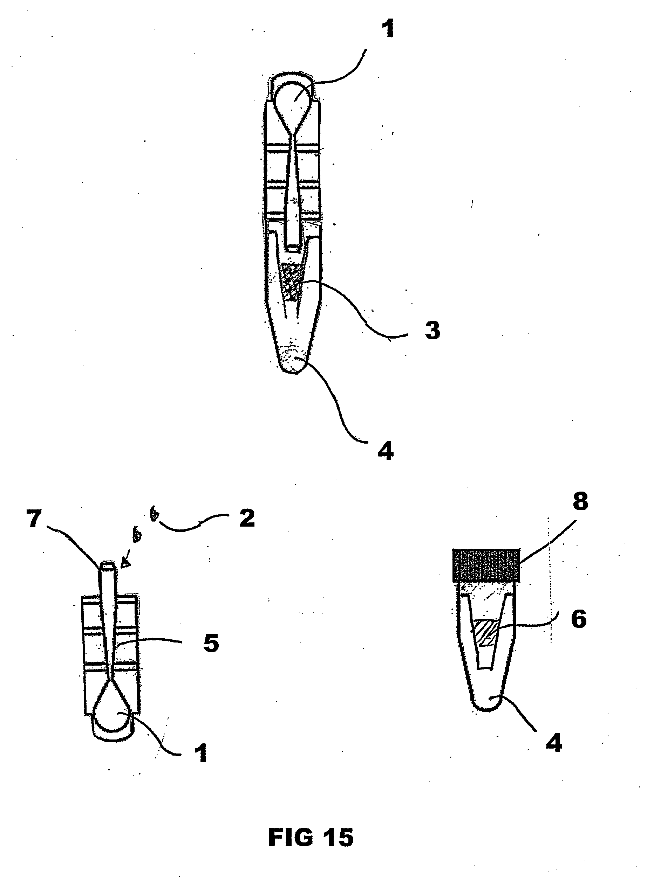

Figur 15 zeigt eine Ausführungsform der Reaktionseinheit.

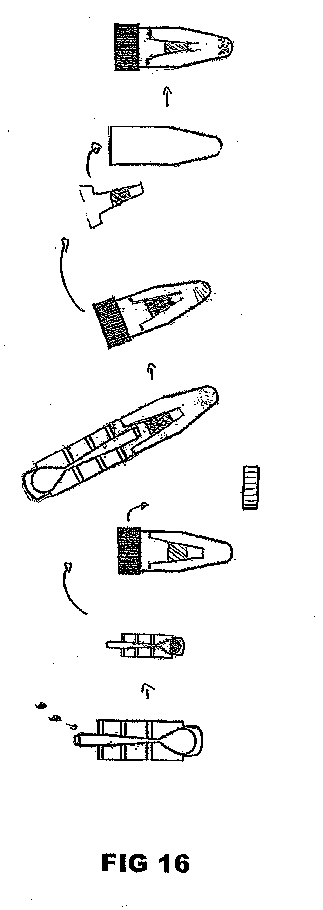

Figur 16 zeigt wie die Reaktionseinheit im beanspruchten Verfahren eingesetzt werden kann.

### Beispiele

### Beispiel 1

Es wurde gefunden, dass sich proteinische bzw. peptidische Aminogruppen mit Aceton und Natriumcyanoborhydrid tatsächlich alkylieren lassen. Diese Reaktion kann sowohl auf ein intaktes Protein als auch auf Peptide einer enzymatischen Hydrolyse eines Protein angewandt werden. Beispielsweise wurde ein tryptischer Verdau des Proteins Glycerinaldehyd-dehydrogenase (GAPH) in wässriger Lösung bei pH ~ 6 mit Aceton und Natriumcyanoborhydrid vermischt und für 12 Stunden stehen gelassen.

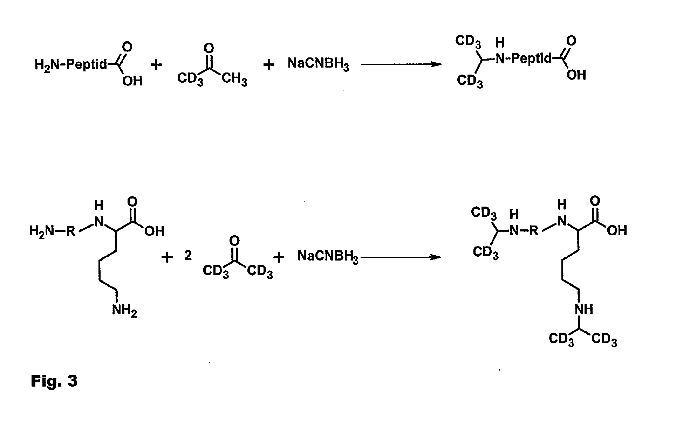

Die massenspektrometische Analyse der Reaktionsprodukte zeigte (Fig. 3), dass die Aminogruppen der Peptide quantitativ und selektiv iso-propyliert wurden. Nebenreaktionen wie Modifizierungen anderer Aminosäuren wurden auch bei langer Reaktionsdauer nicht beachtet. In Peptide, welche neben dem N-Terminus keine weitere primäre Aminogruppe tragen, wurde nur eine iso-Propylgruppe eingebaut (Massenzunahme um 42 Da). In Peptide, welche neben dem N-Terminus eine weitere primäre Aminogruppe wie die ε-Aminogruppe eines C-terminalen Lysinrestes tragen, wurden zwei iso-Propylgruppen eingebaut (Massenzunahme um 84 Da).

### Beispiel 2

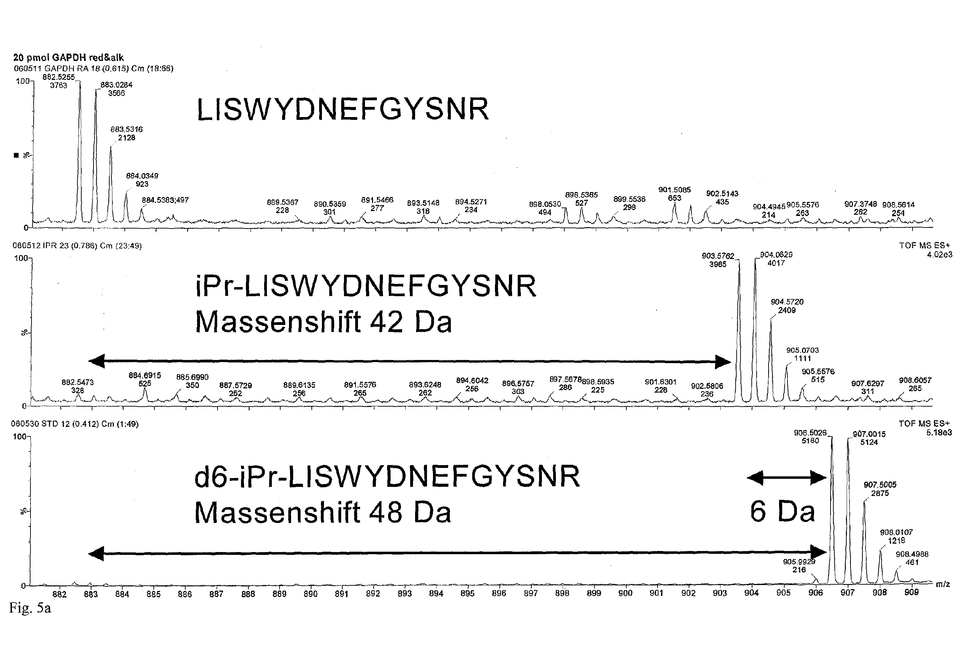

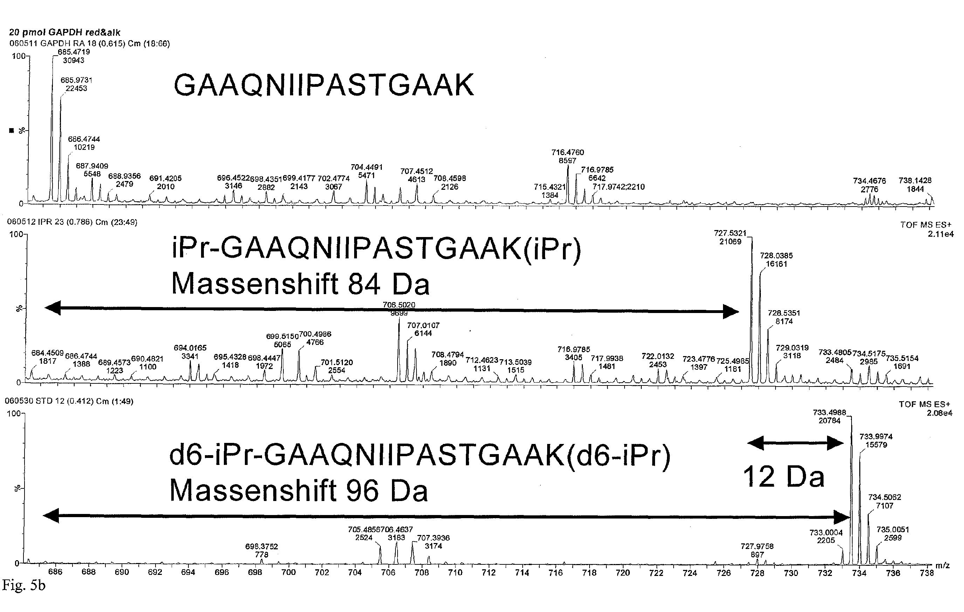

In einem weiteren Experiment wurde ein tryptischer Verdau des Proteins GAPDH in wässriger Lösung bei pH ~ 6 mit D6-Aceton und Natriumcyanoborhydrid vermischt und für 12 Stunden stehen gelassen. Die massenspektrometrische Analyse der Reaktionsprodukte zeigte (Fig. 5), dass die Aminogruppen der Peptide selektiv iso-propyliert wurden, wobei pro iso-Propylrest sechs Deuteriumatome eingebaut wurden. Die Masse von Peptiden, welche neben dem N-Terminus keine weitere primäre Aminogruppe tragen, wurde um 42+6=48 Da erhöht. Die Masse von Peptiden, welche neben dem N-Terminus eine weitere primäre Aminogruppe wie die ε-Aminogruppe eines C-terminalen Lysinrestes tragen, wurde um 84+12=96 Da erhöht. Damit unterscheiden sich nicht-markierte und markierte Peptide um je 6 Da pro iso-Propylrest.

### Beispiel 3

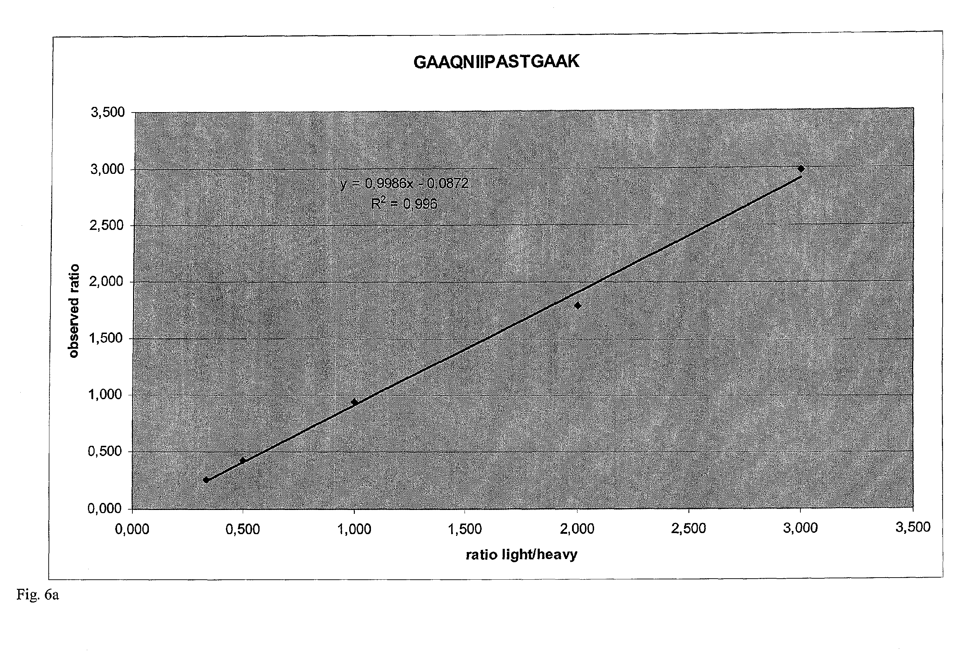

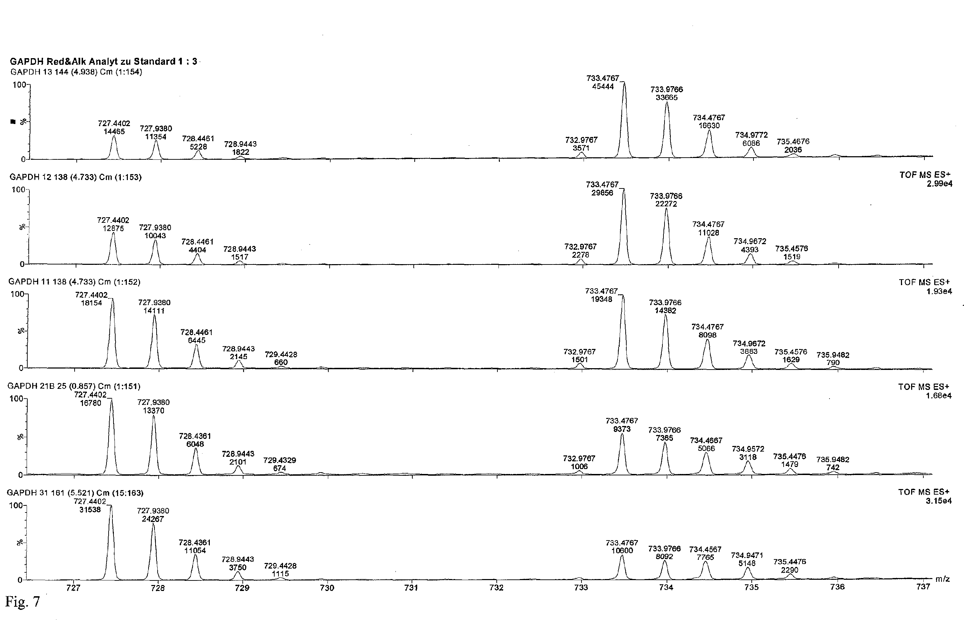

In einem weiteren Experiment wurde ein tryptischer Verdau des Proteins GAPDH in zwei Aliquote gleichen Volumens geteilt. Probe I wurde in wässriger Lösung bei pH ~ 6 mit Aceton und Natriumcyanoborhydrid vermischt und für 12 Stunden stehen gelassen. Probe II wurde analog mit D6-Aceton zur Reaktion gebracht. Nach erfolgter Reaktion wurden die Peptidlösungen in den Verhältnissen 1:3, 1:2, 1:1, 2:1 und 3:1 vermischt und massenspektrometrisch analysiert (Fig. 7). Dabei wurde festgestellt, dass die Verhältnisse der Signalintensitäten von nicht-markierten und markierten Peptiden den eingesetzten Mischungsverhältnissen entsprechen. Als Beispiel sei die Response-Kurve des Peptides GAAQNIIPASTGAAK aufgeführt (Fig. 6), welches in den Formen iPr-GAAQNIIPASTGAAK(iPr) (m/z 727.4 für [M+2H]²⁺) bzw. D6-iPr-GAAQNIIASTGAAK(D6-iPr) (m/z 733.5 für [M+2H]²⁺) vorliegt. Die gemessenen Verhältnisse sind im Bereich von 1:3 bis 3:1 von den eingesetzten Verhältnissen linear abhängig. Durch die nachfolgende Tabelle, sowie Fig. 6, die die Werte graphisch darstellt, ist somit gezeigt, dass zwei Proteinproben quantitativ miteinander verglichen werden können.

**Tabelle 2: Intensitäten der Messsignale von Mischungen eines nicht-markierten und markierten Peptides.**

| Eingesetztes Verhältnis | Intensität m/z 727.4 | Intensität m/z 733.5 | Gemessenes Verhältnis |
|---|---|---|---|
| 0,333 | 11465 | 45444 | 0,252 |
| 0,500 | 12875 | 29856 | 0,431 |
| 1,000 | 18154 | 19348 | 0,938 |
| 2,000 | 16780 | 9373 | 1,790 |
| 3,000 | 31538 | 10600 | 2,975 |

### Beispiel 4

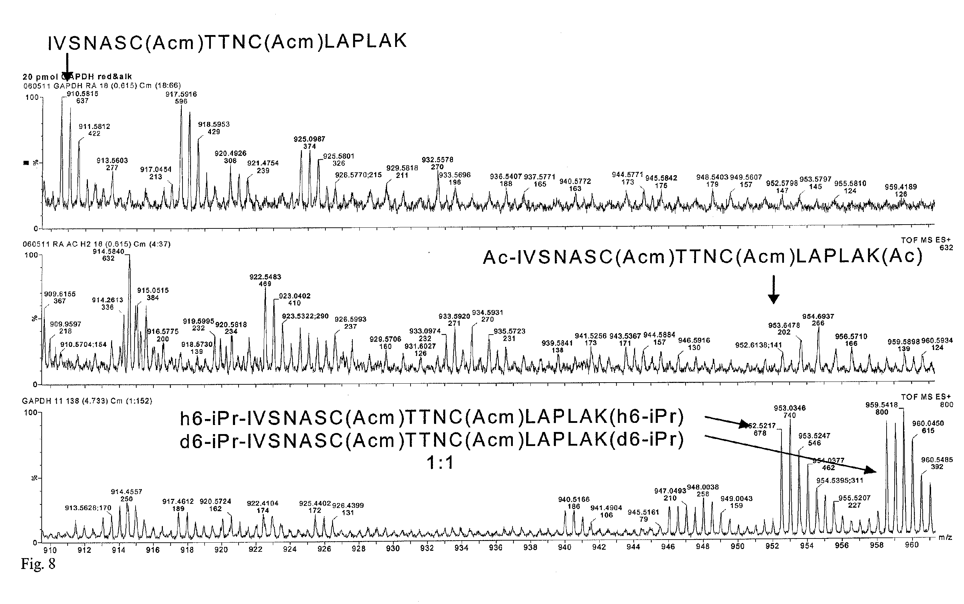

Bei der aufgezeigten Methode bleiben die Ladungsträger der Peptide erhalten. Dadurch wird im Gegensatz zu Methoden, bei denen Aminogruppen acyliert werden, die Ionisierbarkeit von Peptiden nicht eingeschränkt. Beispielsweise wird das im Massenspektrometer sichtbare tryptische Peptid IVSNASC(Acm)TTNC(Acm)LAPLAK aus GAPDH nach Umsetzung mit Essigsäureanhydrid zu Ac-IVSNASC(Acm)TTNC(Acm)LAPLAK(Ac) nicht mehr detektiert, da es keine protonierbaren Gruppen mehr besitzt. Bei Umsetzung mit Aceton und Natriumcyanoborhydrid zum massegleichen iPr-IVSNASC(Acm)TTNC(Acm)LAPLAK(iPr) bleiben die zwei Aminogruppen jedoch protonierbar. Daher bleibt das Peptid im Massenspektrum sichtbar und kann quantifiziert werden (Fig. 8).

### Beispiel 5

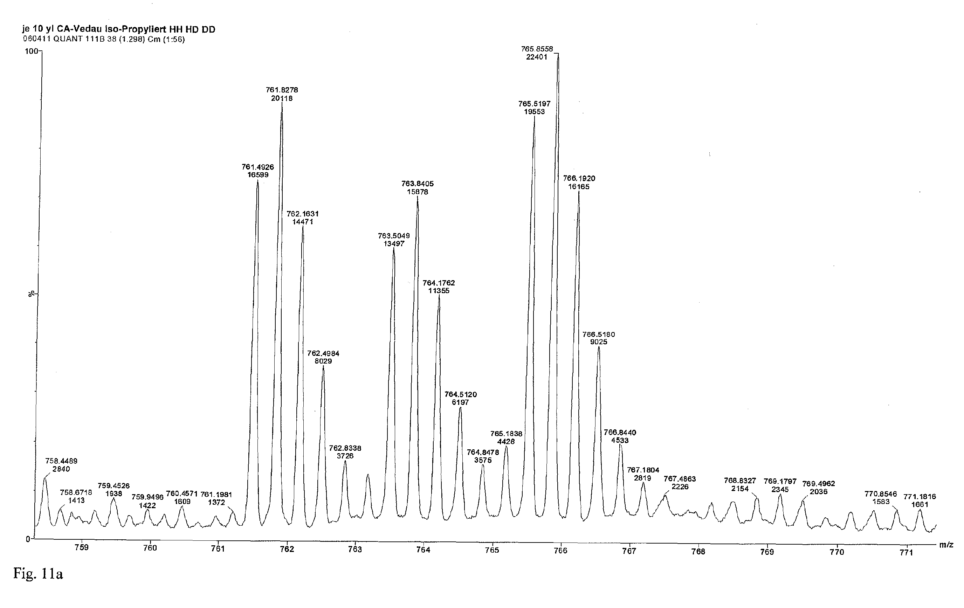

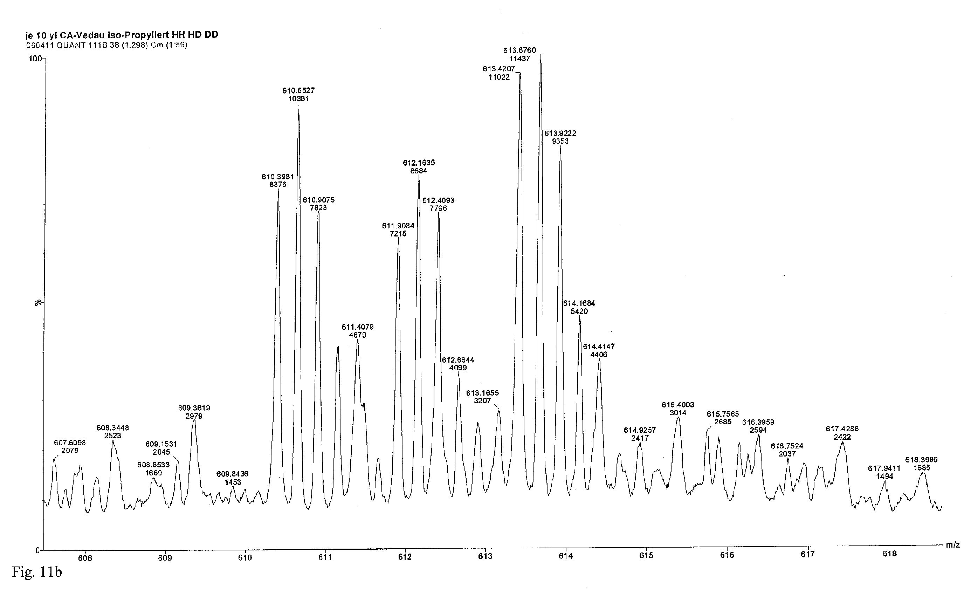

Es wurde ein Verfahren entwickelt, das die parallele Quantifizierung von 3 Proteinproben (I, II und III, jeweils als tryptischer Verdau vorliegend) erlaubt (Fig. 10). Dabei wird die Proteinprobe I bei pH 6 mit Aceton und Natriumcyanoborhydrid iso-propyliert. Die Proteinprobe III wird bei pH 6 mit D6-Aceton und Natriumcyanoborhydrid iso-propyliert. Die Proteinprobe II wird bei zunächst pH 4,75 mit Aceton und Natriumcyanoborhydrid iso-propyliert. Dann werden die Peptide aus II von den Alkylierungsreagenzien befreit und in einem zweiten Schritt bei pH 6 mit D6-Aceton und Natriumcyanoborhydrid iso-propyliert. Wahlweise kann auch bei pH 4 mit D6-Aceton und bei pH 6 mit Aceton gearbeitet werden. Dann werden die Peptidlösungen vereinigt und massenspektrometrisch analysiert. Betrachtet werden im Folgenden nur Peptide mit C-terminalem Lysin. Es entstehen chemisch gleiche Peptide, die sich nur in der Anzahl der eingebauten Deuteriumatome unterscheiden. In die Peptide der Probe I wurden 0, in Probe II 6 und in Probe III 12 Deuteriumatome eingebaut. Dementsprechend kann mittels Massenspektrometrie das ursprüngliche Verhältnis der Proteine I, II und III durch Vergleich der Signalintensitäten ermittelt werden (Fig. 11).

### Beispiel 6

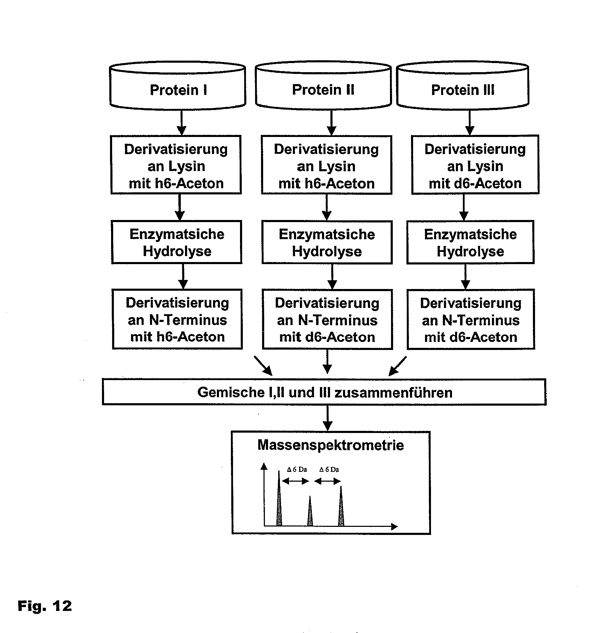

In einer weiteren Variante wird das Labeling vor und nach der enzymatischen Hydrolyse am Protein durchgeführt (Fig. 12). Wird für die beiden Reaktionsschritte ein (bis auf die Isotopenzusammensetzung) identisches Reagenz verwendet, lassen sich wie oben beschrieben drei Proteinproben quantifizieren.

### Beispiel 7

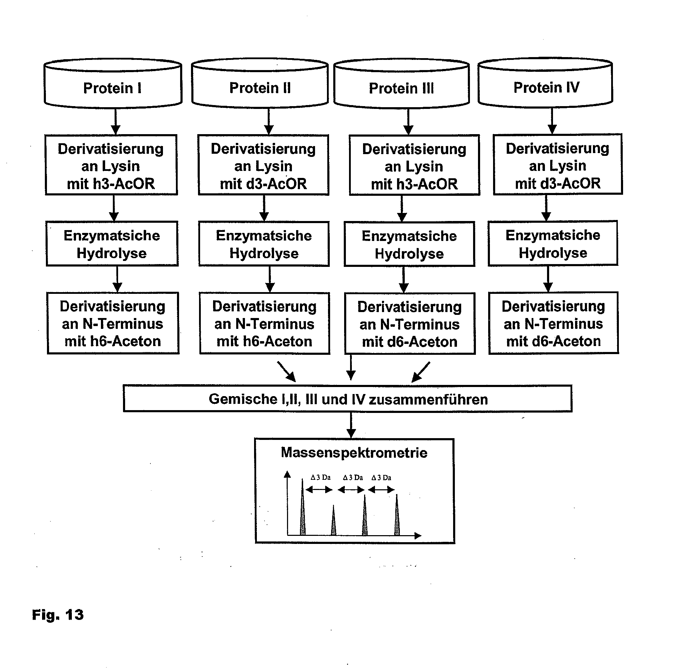

Durch Verwendung von chemisch unterschiedlichen Reagenzien für mehrstufige Reaktion können mehr als drei Proteinproben parallel quantifiziert werden (Fig. 13).

Ein Beispiel für die parallele Quantifizierung von 4 Proteinproben (I, II, III, IV) beruht auf der sequenziellen Anwendung von Acetylierung und Alkylierung (Fig. 14). In einem ersten Reaktionsschritt werden I und III mit aktivierter H3-Essigsäure und II und IV mit aktivierter D3-Essigsäure umgesetzt. Dann wird eine getrennt enzymatische Hydrolyse durchgeführt. I und II werden nun mit H6-Aceton und Natriumcyanoborhydrid, III und IV mit D6-Aceton und Natriumcyanoborhydrid umgesetzt. Es entstehen chemisch gleiche Peptide, die sich nur in der Anzahl der eingebauten Deuteriumatome unterscheiden. In Peptide der Probe I wurden 0, in Probe II 3, in Probe III 6 und in Probe IV 9 Deuteriumatome eingebaut. Dementsprechend kann mittels Massenspektrometrie das ursprüngliche Verhältnis der Proteine I, II, III und IV durch Vergleich der Signalintensitäten ermittelt werden. Auch hier gilt, dass das N-terminale Peptid sowie Lysin-freie Peptide nicht quantifiziert werden können. Ferner zeigen Peptide mit mehrfachen Lysinresten ein abweichendes Isotopomerenmuster. In einer Variation des Verfahrens kann die Reihenfolge Essigsäure - Aceton auch umgedreht werden.

### Beispiel 8

### Acetylierung einer Probe an einer Festphase (Zip Tip C18-Säule).

Dieses Beispiel zeigt die Derivatisierung von ganzen Proteinen oder Peptiden mit Acetanhydrid. Sofern eine Quantifizierung der Protein- oder Peptidproben erfolgen soll, wird eine Probe mit der leichten und die andere Probe mit der schweren isotopischen Form zur Reaktion gebracht.

Grundsätzlich gesprochen wird die H3/D3 Acetylgruppe des Acetanhydrids an die Aminogruppe der Peptide überführt. In Abhängigkeit der Reaktionsparameter kann die N-terminale Aminogruppe des Proteins/Peptids und/oder die Aminogruppen der Lysinreste an den Seitenketten acetyliert werden. Pro Acetylierungsstelle kann eine Massezunahme von 42.106 amu nachgewiesen werden. Die "leichte" (CH3CO-) und die "schwere" (CD3CO-)Acetylgruppen zeigen einen Unterschied von 3 amu. Daher kann die Zahl der eingeführten Acetylgruppen an der Massendifferenz des einzelnen Paares abgelesen werden. Acetanhydrid wie auch N-Acetoxysuccinimid sind in wässrigem Milieu hochreaktiv mit Hydroxylgruppen. Hauptsächlich Serin, Threonin und Thyrosin sind hiervon betroffen. Die Bildung dieser Ester kann jedoch mit N-Hydroxylamin umgekehrt werden. Die Acetatester können in wenigen Minuten bei basischem pH hydrolysiert werden.

Für die Acetylierung an der Säule wird eine Zip Tip C18 Pipettenspitze durch dreimaliges Aufziehen und Verwerfen von 20 µl einer 100 %-igen Acetonitrillösung und einem einmaligen Aufziehen und Verwerfen mit einer wässrigen Lösung von 0,1 % Ameisensäure aktiviert. Um eine vollständige Bindung des Proteins oder der Peptide an die C18 Matrix in der Zip Tip Mikrosäule zu garantieren, werden die angesäuerten Peptidlösungen in Fraktionen geteilt und langsam mehrere Male auf-und abpipettiert. Alternativ können die Peptidfraktionen auch auf die Säule aufzentrifugiert werden. Die angesäuerte Peptidlösung enthält dabei 0,2 % Ameisensäure. Die Acetylierung auf der Säule wurde anschließend wie folgt ausgeführt. Die Acetylierungslösungen wurden frisch für jede Probe hergestellt. Um die Lysinseitenketten zu acetylieren und die N-terminalen Aminogruppen der Peptide zu acetylieren, wurden 9 µl einer 6 M Guanidin x HCL und 1 M Hepes (pH 8,5) in einem Verhältnis 10 : 1 (v/v) und mit 1 µl einer 1 % (v/v) Acetanhydridlösung (H6/D6) in Acetonitril gemischt (Lösung A). Das Acetanhydrid kommt dabei in zwei isotopen Formen zur Anwendung, nämlich entweder als H6 mit einer Masse von 102 g/mol oder als D6 mit einer Masse von 108 g/M. Abschließend wurde ein Volumen von 5 µl der Lösung A in die Pipettenspitze aufgezogen bzw. auf die Säule aufzentrifugiert und die Lösung auf der stationären Phase des Zip Tips belassen. Die Acetylierung wurde für 3 Minuten bei Raumtemperatur durchgeführt.

Die Acetylierungsreaktion wurde mit 6 µl einer wässrigen Abstopplösung, enthaltend 67 µM Hydroxylamin und 67 µM NaOH (Lösung B), abgestoppt. Dieses Reagenz hydrolisiert auch die Ester (O-Acetylierungen) der Aminosäuren Serin, Threonin und Thyrosin, die während der N-Acetylierung der Seitenketten gebildet wurden. Weitere 5 ml der Lösung B wurden auf die Säule gegeben bzw. in die Säule pipettiert und anschließend die Deacetylierung für 10 Minuten bei Raumtemperatur durchgeführt. Die C18 Matrix wurde dann 10 mal mit 20 µl einer 0,1 %-igen (v/v) Ameisensäure gewaschen, indem die Lösung auf die Säule aufgetragen und hindurchzentrifugiert wird oder durch die Pipettenspitze aufgezogen und ausgeworfen wird. Abschließend werden die chemisch umgesetzten Peptide aus der Mikrosäule mit 3 µl einer 65 % Acetonitril, 1 % 2-Propanol und 0,1 % Ameisensäure (v/v/v) enthaltenen Lösung ausgewaschen und bei 4° Celsius bis zur massenspektroskopischen Analyse aufbewahrt.

Die Derivatisierung auf der Säule von Peptiden mit Acetanhydrid wirft ein Problem auf. Zum ersten zeigt Acetanhydrid eine niedrige Löslichkeit in Wasser, so dass sich die Lösungen in eine untere Phase aus Ac₂O und eine wässrige obere Phase trennen. Es zeigte sich, dass diese Phasentrennung für eine Acetylierung auf der Säule ungeeignet ist, da das konzentrierte Ac₂O die Zip Tip (Millipore) Matrix zerstört. Acetanhydrid ist jedoch in Acetonitril löslich, was die Löslichkeit von geringen Konzentrationen des Acetanhydrids in wässrigen Lösungen begünstigt. Aus diesem Grund wurde die Endkonzentration des Acetonitrils und Acetanhydrids zur Derivatisierung der Amine mit 10 % und 0,1 % (v/v) gewählt. Diese Konzentration von Acetonitril beeinflusst die Peptidbindung in der Matrix nicht, da normalerweise die Peptide mit einer Konzentration von größer 15 % Acetonitril eluiert werden.

## Patentansprüche

**1.** Verfahren zur quantitativen Analyse von zwei, drei oder mehreren Protein- oder Peptidproben, enthaltend wenigstens die folgenden Verfahrensschritte:
• eine erste Derivatisierungsreaktion in der die Aminogruppe am N-terminale Ende von wenigstens einer der besagten Protein- oder Peptidproben voneinander unabhängig alkyliert wird;
• eine zweite Derivatisierungsreaktion in der andere Aminogruppen als die am N-terminale Ende, vorzugsweise wenigstens eine Aminogruppe von wenigstens einer Seitenkette von wenigstens einer der besagten Protein- oder Peptidproben unabhängig von der ersten Derivatisierungsreaktion alkyliert wird;
• einer vor, nach oder zwischen den Derivatisierungsreaktionen erfolgenden enzymatischen Hydrolysierungsreaktion von wenigstens einer der besagten Protein- oder Peptidproben;
• einer analytischen Messung der besagten Protein- oder Peptidproben mittels Massenspektrometrie,
wobei wenigstens eine Derivatisierungsreaktion eine reduktive Alkylierungen mit einem Keton in Anwesenheit eines Hydrierungsreagenz ist, wobei, sofern mehr als zwei Protein-oder Peptidproben quantifiziert werden, zusätzlich ein und/oder mehrere stabilisotopenmarkierte Analoga des Ketons bzw. des Hydrierungsreagenz in verschiedenen Kombinationen eingesetzt werden.

**2.** Verfahren nach Anspruch 1,
**dadurch gekennzeichnet, dass**
in der ersten und/oder zweiten Derivatisierungsreaktion als Keton Aceton und/oder ein Acetonderivat bzw. stabilisotopenmarkiertes Aceton und/oder ein stabilisotopenmarkiertes Acetonderivat eingesetzt werden.

**3.** Verfahren nach einem der vorangegangenen Ansprüche,
**dadurch gekennzeichnet, dass**
das eingesetzte Hydrierungsreagenz Natriumcyanoborhydrid oder Natriumcyanobordeuterid enthält.

**4.** Verfahren nach einem der vorangegangenen Ansprüche,
**dadurch gekennzeichnet, dass** wenigstens einer der folgenden Verfahrensschritte eingeschlossen ist:
• ein Trennschritt wie z.B. Gelelektrophorese und/oder Chromatographie zur Herabsetzung der Komplexität der Probe, indem die Probe in Fraktionen aufgeteilt wird;
• ein Abtrennungsschritt zur Entfernung von unspezifisch oder unvollständig markierten Peptiden oder Proteinen;
• eine Zusammenfassung der zu analysierenden Protein- oder Peptidproben zu einem Gemisch.

**5.** Verfahren nach einem der vorangegangenen Ansprüche
**dadurch gekennzeichnet, dass**
eine oder mehrere der Protein- oder Peptidproben in der ersten Derivatisierungsreaktion mit einem ersten Derivatisierungsreagenz abgeleitet von Aceton oder einer stabilisotopenmarkierten Verbindung des Acteons und in der zweiten Derivatisierungsreaktion mit einem zweiten, vom Aceton verschiedenen Derivatisierungsreagenz zur Reaktion gebracht werden.

**6.** Verfahren nach Anspruch 5,
**dadurch gekennzeichnet, dass**
die Derivatisierungen mit Aceton und dem vom Aceton verschiedenen Derivatisierungsreagenz bzw. deren Derivaten in beliebiger Reihenfolge durchgeführt werden.

**7.** Verfahren nach einem der vorangegangenen Ansprüche,
**dadurch gekennzeichnet, dass**
vorzugsweise als stabilisotopenmarkierte Verbindungen in der ersten und/oder zweiten Derivatisierungsreaktion D6-Aceton und/oder aktivierte D3-Essigsäure eingesetzt werden.

**8.** Verfahren nach einem der vorangegangenen Ansprüche,
**dadurch gekennzeichnet, dass**
die Reaktionsbedingungen derart gewählt sind, dass in der ersten Derivatisierungsreaktion nur die N-terminalen Aminogruppen der zu analysierenden Proteine oder Peptide reduktiv alkyliert werden.

**9.** Verfahren nach Anspruch 8,
**dadurch gekennzeichnet, dass**
die Derivatisierungsreaktion der Aminogruppe der N-terminalen Enden von einer oder mehreren der besagten Protein- oder Peptidproben bei einem pH-Wert von ≤ 5 durchgeführt wird.

**10.** Verfahren nach einem der vorangegangenen Ansprüche,
**dadurch gekennzeichnet, dass**
die Reaktionsbedingungen derart gewählt sind, dass in der zweiten Derivatisierungsreaktion neben bzw. außer den Aminogruppen der N-Termini auch andere Aminogruppen der zu analysierenden Proteine oder Peptide alkyliert werden.

**11.** Verfahren nach Anspruch 10,
**dadurch gekennzeichnet, dass**
die Derivatisierungsreaktion von wenigstens einer Aminogruppe von einer oder mehrerer der besagten Protein- oder Peptidproben bei einem pH-Wert von ≥ 5 durchgeführt wird.

**12.** Verfahren nach einem der vorangegangenen Ansprüche,
**dadurch gekennzeichnet, dass**
vor den Derivatisierungsreaktionen oder vor der analytischen Massenspektroskopie zumindest eine teilweise Auftrennung des Proteingemisches mittels ein- und/oder zweidimensionaler Gelelektrophorese oder Chromatographie durchgeführt wird.

**13.** Verfahren nach einem der vorangegangenen Ansprüche,
**dadurch gekennzeichnet, dass**
die erste und/oder zweite Derivatisierungsreaktion und/oder Hydrierungsreaktion der Peptid- oder Proteinprobe, an oder in einer Festphase oder Matrix gebunden oder zurückgehalten, stattfinden.

**13.** Verfahren nach Anspruch 12,
**dadurch gekennzeichnet, dass**
es sich bei der Festphase oder Matrix um ein Sorbens, insbesondere Polyacrylamid, Kieselgel, modifiziertes Kieselgel oder einen Ionenaustauscher handelt.

**15.** Reaktionseinheit zur Durchführung des Verfahrens nach einem der vorangegangenen Ansprüche,
**dadurch gekennzeichnet, dass**
das Reaktionsgefäß einen ersten Reaktionsraum aufweist, welcher der Aufnahme der Protein- oder Peptidprobe, z.B. in Lösung oder in einer Matrix-Struktur eines Trägermaterials aufgenommenen, dient und
einen zweiten Reaktionsraum aufweist, welcher der Aufnahme der Protein- oder Peptidprobe zur Durchführung wenigstens der ersten und/oder der zweiten Derivatisierungsreaktion und/oder Hydrolysierungsreaktion dient und
einen dritten Reaktionsraum aufweist, welcher der Aufnahme der chemisch umgesetzten Protein- oder Peptidprobe dient,
wobei der erste Reaktionsraum mit dem zweiten Reaktionsraum durch einen Übergangsbereich verbunden ist, der einen sich verengenden Querschnitt aufweist, welcher durch wenigstens ein bezüglich einer Längsachse der Reaktionseinrichtung zumindest teilweise geneigt verlaufendes Begrenzungselement bestimmt wird, und
wobei der zweite Reaktionsraum mit dem dritten Reaktionsraum durch einen Übergangsbereich verbunden ist, der derart ausgeführt ist, dass eine Festphase oder Matrix, in oder an welcher wenigstens die erste und/oder die zweite Derivatisierungsreaktion und/oder die Hydrolysierungsreaktion durchgeführt wird, in dem Übergangsbereich angeordnet ist.
